(19) Europäisches Patentamt
European Patent Office
Office européen des brevets

(11) Veröffentlichungsnummer: **0 189 801**
**B1**

(12) # EUROPÄISCHE PATENTSCHRIFT

(45) Veröffentlichungstag der Patentschrift: **25.01.89**

(51) Int. Cl.⁴: **C 07 D 311/92,** A 61 K 31/35, C 12 P 17/02, C 12 P 17/06

(21) Anmeldenummer: **86100633.6**

(22) Anmeldetag: **18.01.86**

(54) **Verfahren zur Herstellung von Forskolinderivaten mittels mikrobieller Umwandlungen.**

(30) Priorität: **26.01.85 DE 3502685**

(43) Veröffentlichungstag der Anmeldung: **06.08.86 Patentblatt 86/32**

(45) Bekanntmachung des Hinweises auf die Patenterteilung: **25.01.89 Patentblatt 89/4**

(84) Benannte Vertragsstaaten: **AT BE CH DE FR GB IT LI NL SE**

(56) Entgegenhaltungen:
**EP-A-0 116 713**
**WO-A-85/02616**
**DE-A-2 557 784**
**DE-A-2 654 796**
**DE-A-3 314 999**
**US-A-4 476 140**

(73) Patentinhaber: **HOECHST AKTIENGESELLSCHAFT, Postfach 80 03 20, D-6230 Frankfurt am Main 80 (DE)**

(72) Erfinder: **Khandelwal, Yatendra, Dr., 10C/10 NityaNand Nagar 80/83 R.C. Marg, Chembur Bombay 400 071 (IN)**
Erfinder: **Chatterjee, Sukumar, Dr., G-32 Hoechst Quarters, Mulund (West) Bombay 400 082 (IN)**
Erfinder: **Nadkarni, Suresh Rudra, Dr., 5 Yagneshwar Kasturba Marg, Mulund (West) Bombay 400 080 (IN)**
Erfinder: **Ganguli, Bimal Naresh, Dr., 173 Central Avenue Chembur, Bombay 400 071 (IN)**
Erfinder: **Inamdar, Prabhakar Krishnaji, Dr., 7, Vijaya Sadan Scheme 6 Road No. 25, Sion (West) Bombay 400 022 (IN)**
Erfinder: **Dohadwalla, Alihussein N., Dr., Moman Mansion 139 C, Cumbala Hill, Bombay 400 036 (IN)**
Erfinder: **Rupp, Richard Helmut, Dr., A84, Meher Apartments Anstey Road, Off Altamount Road Bombay 400 026 (IN)**
Erfinder: **de Souza, Noel John, Dr., 702 Avanti Central Avenue, Santacruz (West) Bombay 400 054 (IN)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Die Erfindung betrifft ein Verfahren zur Herstellung von Forskolinderivaten mittels mikrobieller Umwandlungen. Die so erhaltenen Substanzen können als pharmazeutische Mittel, insbesondere als Medikamente zur Behandlung von Glaukom, Herzinsuffizienz, Hypertonie, Entzündung, Allergien, Bronchialerkrankungen und Erkrankungen des Immunsystems eingesetzt werden.

Forskolin und seine natürlich vorkommenden Analoge stellen eine Gruppe von Labdan-Terpenoiden dar, die aus der Pflanze Coleus forskohlii (Briqu.) isoliert werden. Die Srukturformeln von Forskolin und seinen natürlich vorkommenden, isolierten Analogen werden in der Formel I dargestellt.

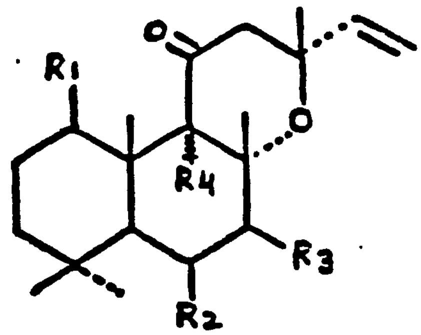

Für Forskolin ist $R_1 =$ 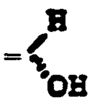 $R_2 =$ 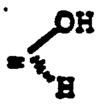 $R_3 =$ 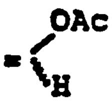 und $R_4 =$ OH

Bis heute sind folgende natürlich vorkommende Forskolin-Analoge nachgewießen worden:

1,9-Didesoxy-forskolin: $R_1 = H_2$, $R_2 =$ 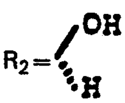, $R_3 =$ 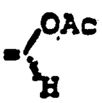 und $R_4 =$ H;

9-Desoxy-forskolin: $R_1 =$ 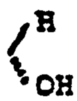, $R_2 =$ 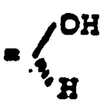, $R_3 =$ 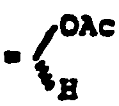, $R_4 =$ H;

1,9-Diesoxy-7-deacetyl-forskolin: $R_1 = H_2$, $R_2, R_3 =$ 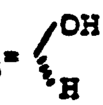, $R_4 =$ H;

1-Desoxy-forskolin: $R_1 = H_2$, $R_2 =$ 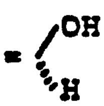, $R_3 =$ 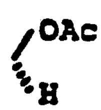, $R_4 =$ OH;

7-Deacetyl-forskolin: $R_1 =$ 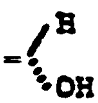, $R_2 = R_3 =$ 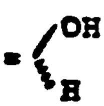, $R_4 =$ OH:

6-Acetyl-7-deacetyl-forskolin: $R_1 =$ 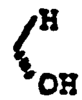, $R_2 =$ 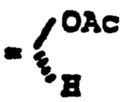, $R_3 =$ 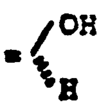, $R_4 =$OH

(Tetrahedron Lett. 19, (1977) 1669; J.Med.Chem. 26, (1983) 486.

Forskolin ist eines der wichtigen Diterpene der Pflanze Coleus Forskohlii, und kann verwendet werden als wertvolles Arzneimittel bei der Behandlung verschiedener Erkrankungen wie Glaukom, Hypertonie, Herzinsuffizienz, Entzündung, Allergien, Bronchokonstriktion und ganz allgemein von Erkrankrungen, die durch Störungen der cAMP-Produktion und -Metabolismus hervorgerufen werden (DE-OS-2 557 784, Indisches Patent Nr. 147 630 und entsprechende DE-DS-2 640 275; Med.Res.Revs. 3, (1983) 201-19). Von allen bekannten Forskolin-Analogen mit ähnlichen biologischen Eigenschaften (DE-AS-2 640 275, EP-A-116 713 und DE-OS-2 654 796) ist Forskolin zur Zeit die für die klinische Anwendbarkeit bevorzugt Substanz (DE-OS-3 314 999).

Ein Verfahren zur Isolierung des Hauptanteil von Forskolin aus Coleus forskohlii ist bereits beschrieben (s. DE-OS-2 557 784). Bei diesem Verfahren werden außerdem Forskolin-Analoge gewonnen. Diese Analoge

können verwendet werden als Zwischenprodukte zur Herstellung von Forskolin. Es sind bereits Methoden beschrieben worden, durch die die Analoge mit gleicher Oxidationsstufe wie Forskolin, z. B. 7-Deacetyl-Forskolin und 6-Acetyl-7-Deacetylforskolin in Forskolin umgewandelt werden können (DE-OS-2 654 796, J. Chem. Soc. Perk. Trans. 1, (1982) 767).

Gegenstand der vorliegende Erfindung ist nun ein Verfahren die mikrobiellen Umwandlung, durch das weniger hydroxylierte Forskolin-Analoge in 7-Deacetyl-Forskolin oder 6-Acetyl-7-deacetylforskolin, oder in solche Derivate umgewandelt werden, die sich durch bekannte chemische methoden leicht in Forskolin umwandeln lassen.

Ein weiterer Aspekt der Erfindung ist es die Mikroorgansismen zu beschreiben, die die Umwandlung bewirken, sowie die Art und Weise, in der sie in dem Verfahren der mikrobiellen Umwandlung von Forskolinanalogen verwendet werden. Es sind mikrobielle Umwandlungen von Verbindungen beschrieben worden, die zur Gruppe der Steroide gehören (L.F. Fieser und M. Fieser "Steroide", Asia Publishing House, (1962) S. 672-75). Unseres Wissens sind derartige Umwandlungen bei Forskolin-Derivaten bisher nicht angewandt worden.

Die erfindungsgemäß erhaltenen Substanzen können als Medikamente zur Behandlung von Glaukom, Hypertonie, Herzinsuffizienz, Entzündungen, Allergien, Bronchialerkrankungen und von Erkrankungen, die durch das Immunsystem beeinflußt werden, verwendet werden.

Gegenstand der Erfindung ist somit ein Verfahren zur Herstellung von Forskolinderivaten, das dadurch gekennzeichnet ist, daß man auf ein bestimmtes Forskolinderivat als Substrat in einem Nährmedium auf übliche Weise einen bestimmten Mikroorganismus einwirken läßt und die gebildete Verbindung nach Beendigung der Fermentation aus der Kulturbrühe und dem Mycel durch Extraktion mit einem organischen Lösungsmittel und anschließende Chromatographie und/oder Kristallisation isoliert, wobei man

a) aus der Verbindung der Formel III

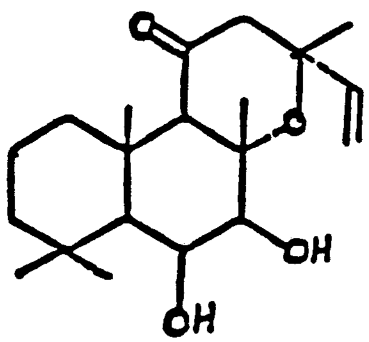

III

als Substrat und unter Verwendung des Stammes H/134 (DSM 3205) die Verbindung der Formel IV

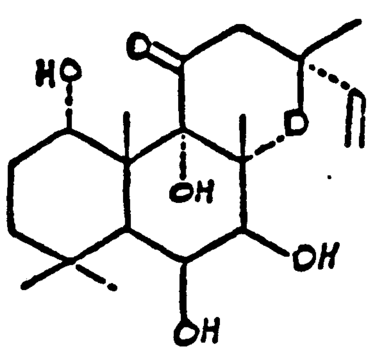

IV

erhält,

b) aus der Verbindung der Formel IV als Substrat und unter Verwendung des Stammes FF/406 (DSM 3211) die Verbindung der Formel V

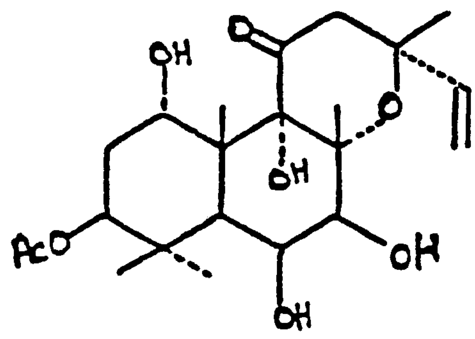

V

worin Ac den Rest $CH_3CO$- bedeutet, erhält,
c) aus der Verbindung der Formel VI

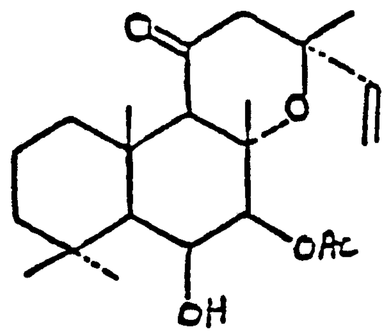

VI

worin Ac den Rest $CH_3CO$- bedeutet, als Substrat und
$c_1$) unter Verwendung des Stammes FF/172 (DSM 3208) die Verbindung der Formel VII

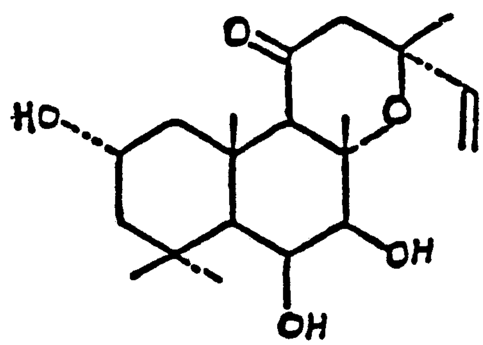

VII

oder
$c_2$) unter Verwendung des Stammes FF/101 (DMS 3207) die Verbindung der Formel VIII

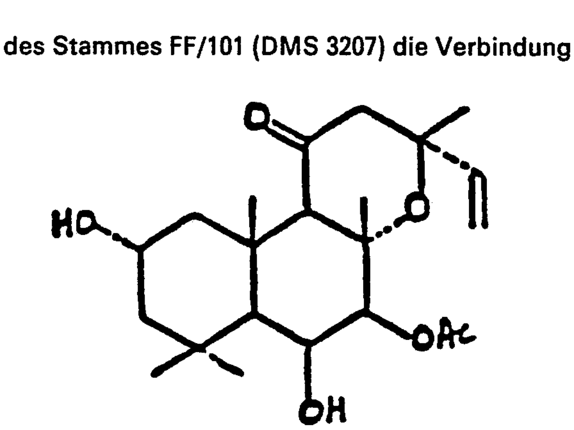

VIII

worin Ac den Rest $CH_3CO$- bedeutet,
oder
$c_3$) unter Verwendung des Stammes H/134 (DSM 3205) die Verbindung der Formel IX

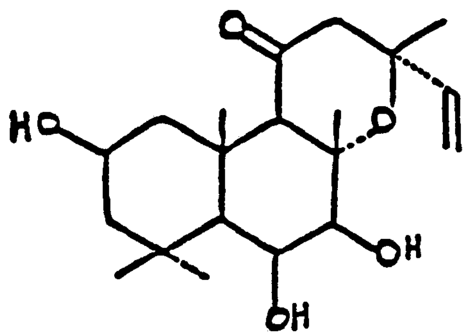

IX

oder

$c_4$) unter Verwendung des Stammes FF/101 (DSM 3207) die Verbindung der Formel X

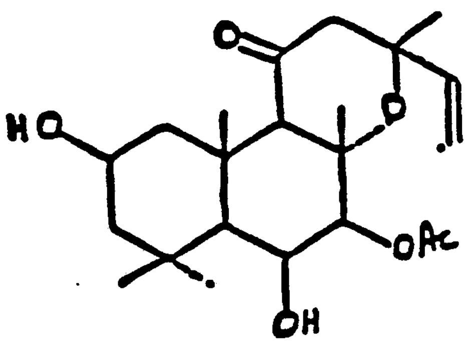

X

worin Ac den Rest $CH_3CO$- bedeutet,
oder

$c_5$) unter Verwendung des Stammes H/134 (DSM 3205) die Verbindung der Formel XI

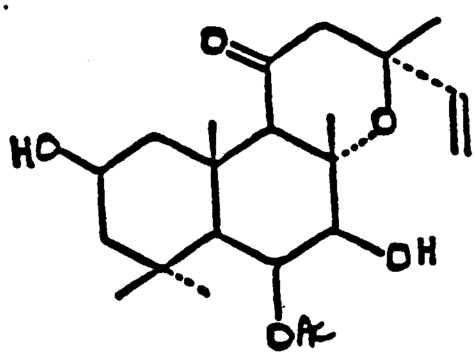

XI

worin Ac den Rest $CH_3CO$- bedeutet,
oder

$c_6$) unter Verwendung des Stammes FF/173 (DSM 3209) oder H/1 (DSM 3202) die Verbindung der Formel XII

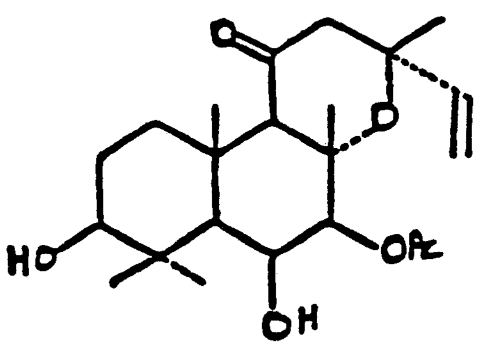

XII

worin Ac den Rest $CH_3CO$- bedeutet,
oder

$c_7$) unter Verwendung des Stammes FF/383 (DSM 3210) die Verbindung der Formel XIII

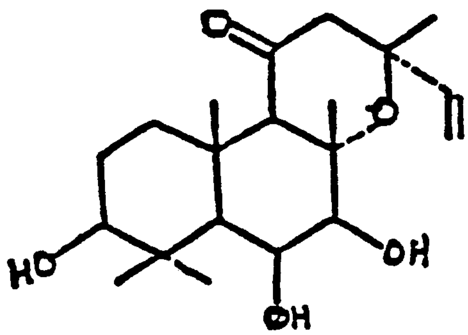

XIII

oder

$c_8$) unter Verwendung des Stammes FF/695 (DSM 3212) die Verbindung der Formel XIV

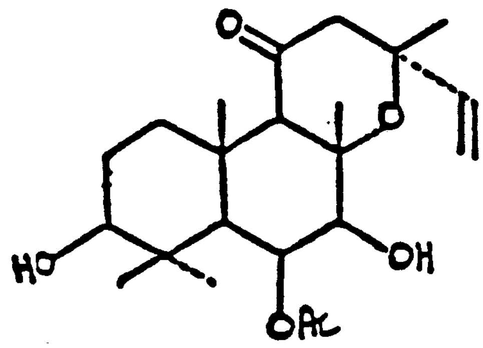

XIV

worin Ac den Rest $CH_3CO$- bedeutet,

oder

$c_9$) unter Verwendung des Stammes FF/93 (DSM 3206) oder FF/172 (DSM 3208) die Verbindung der Formel XV

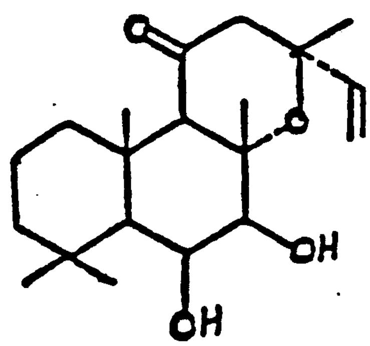

XV

oder

$c_{10}$) unter Verwendung des Stammes H/3 (DSM 3203) oder H/19 (DSM 3204) oder Y-82 52 904 (DSM 3213) die Verbindung der Formel XVI

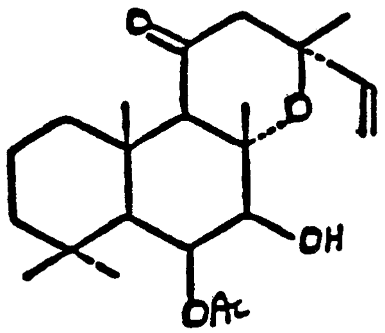

XVI

worin Ac den Rest $CH_3CO$- bedeutet,
oder
$c_{11}$) unter Verwendung des Stammes H/1 (DSM 3202) die Verbindung der Formel XVII

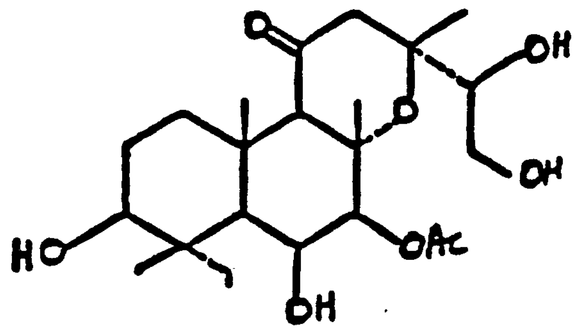

XVII

worin Ac den Rest $CH_3CO$- bedeutet, erhält.

Ein besonderes Merkmal der Erfindung besteht darin, daß die erfindungsgemäße Umwandlung von Forskolinderivaten durch mikrobielle Stämme erfolgt, die nach konventionellen Methoden aus Bodenproben gewonnen werden. Die Stämme, die diese Umwandlungen bewirken, sind nachstehend in Tabelle I aufgeführt und, wie angegeben, identifiziert worden.

**TABELLE 1**

| Stamm-Nr. | Speizes |
|---|---|
| H/1 (DSM 3202) | Aspergillus niger |
| H/3 (DSM 3203) | noch nicht bestimmt |
| H/19 (DSM 3204) | noch nicht bestimmt |
| H/134 (DSM 3205) | Scopulariopsis sp. |
| FF/93 (DSM 3206) | noch nicht bestimmt |
| FF/101 (DSM 3207) | Syncephalastrum sp. |
| FF/172 (DSM 3208) | noch nicht bestimmt |
| FF/173 (DSM 3209) | noch nicht bestimmt |
| FF/383 (DSM 3210) | Aspergillus niger |
| FF/406 (DSM 3211) | noch nicht bestimmt |
| FF/695 (DSM 3212) | noch nicht bestimmt |
| I-8252904 (DSM 3213) | Pseudomonas species |

Die Stämme wurden unter den oben genannten Nummern bei der Deutschen Sammlung von Mikroorganismen, GÖttingen, hinterlegt am 22.1.1985.

In der nachfolgenden Tabelle II werden die bei den Unwandlungen verwendeten Substrate, die Stämme, die diese Umwandlungen bewirken, die erzeugten Produkte und deren Schmelzpunkke aufgeführt.

**TABELLE II**

| Substanz-Nr | Substrat | Stamm | Produkt | Schmelzpunkt °C | Beispiel Nr. |
|---|---|---|---|---|---|
| 1 | 7-Deacetyl-1,9-didesoxyforskolin (Fig. 12) | H/134 | 7-Deacetylforskolin (Fig. 3) | 177-178 | 1 |
| 2 | 7-Deacetylforskolin (Fig. 3) | FF/406 | 3β-Acetoxy-7 deacetylforskolin (Fig, 2) | 256-258 | 2 |
| 3 | 1,9-Didesoxyforskolin (Fig. 1) | FF/172 | 7-Deacetyl-2α-hydroxy-1,9-didesoxy-forkolin (Fig. 4) | 86-88 | 3 |
| 4 | 1,9-Didesoxyforskolin (Fig. 1) | FF/101 | 2α-Hydroxy-1,9-didesoxyforskolin (Fig. 5) | 209-211 | 4 |
| 5 | 1,9-Didesoxyforskolin (Fig. 1) | H/134 | 7-Deacetyl-2β-hydroxy-1,9-didesoxyforskolin (Fig. 6) | 84-86 | 5 |
| 6 | 1,9-Didesoxyforskolin (Fig. 1) | FF/101 | 2β-Hydroxy-1,9-didesoxyforskolin (Fig. 7) | 258-61 | 6 |
| 7 | 1,9-Didesoxyforskolin | H/134 | 6β-Acetyl-7-deacetyl-2β-hydroxy-1,9-didesoxyforskolin (Fig. 8) | 168-70 | 7 |
| 8 | 1,9-Didesoxyforskolin (Fig. 1) | FF/383 FF/173 H/1 | 3β-Hydroxy-1,9-didesoxyforskolin (Fig. 9) | 271-272 | 8 |
| 9 | 1,9-Didesoxyforskolin (Fig. 1) | FF/383 | 7-Deacetyl-3β-hydroxy-1,9-didesoxyforskolin (Fig. 10) | 78-80 | 9 |
| 10 | 1,9-Didesoxyforskolin (Fig. 1) | FF/695 | 6β-Acetyl-7-deacetyl-3β-hydroxy-1,9-didesoxyforskolin (Fig. 11) | 200-202 | 10 |
| 11 | 1,9-Didesoxyforskolin (Fig. 1) | FF/93 F/172 | 7-Deacetyl-1,9-didesoxyforskolin (Fig. 12) | 121-122 | 11 |
| 12 | 1,9-Didesoxyforskolin (Fig. 1) | H/3 H/19 | 6-β-Acetyl-7-deacetyl-1,9-didesoxyforskolin (Fig. 13) | 149 | 12 |
| 13 | 1,9-Dideosoxyforskolin (Fig. 1) | H/1 | 3β-14,15-trihydroxy-1,9-didesoxyforskolin (Fig. 14) | Öl | 13 |

Das erfindungsgemäße Verfahren ist gekennzeichnet durch die Fermentierung eines bestimmten Forskolinderivats in einem üblichen Nährmedium definierter Zusammensetzung, das einen der obene erwähnten Mikroorganisem-Stämme (wie in Tabelle I aufgeführt) für jedes Substrat enthält; die Fermentierung erfolgt 8 bis 10 Tage lang bei einer Temperatur von 28 bis 30°C.

Das Nährmedium enthält Kohlenstoff- und Stickstoffquellen, gegebenenfalls auch anorganische Nährsalze sowie Spurenelemente.

Als Kohlenstoffquellen eignen sich z. B. Glucose, Stärke, Dextrin und Glycerin. Geeignete Stickstoffquellen sind z. B. Sojamehl, Hefeextrakt, Fleischextrakt, Malzextrakt, Maisquellwasser, Pepton und Casein. Als anorganieche Nährsalze kommen z. B. Natriumchlorid, Magnesiumsulfat oder Calciumcarbonat, als Spurenelemente z. B. Eisen, Magnesium, Kupfer, Zink und Kobalt in Form geeigneter Salze in Frage.

Die Kulturbrühe wird filtriert und daß Mycel und das Filtrat getrennt mit organischen Lösungsmitteln wie Methylenchlorid, Chloroform oder Äthylacetat extrahiert. Die Extrakte werden vereinigt, über Trocknungsmitteln wie $Na_2SO_4$ getrocknet und im Vakuum konzentriert. Der Rückstand wird dann durch Trennungsverfahren wie beispielsweise Chromatographie und durch Kristallisation gereinigt. Die Strukturen in den Produkten auf der Basis ihrer Spektral- und Elementaranalyse zugeordnet worden.

Die erfindungsgemäß erhaltenen Verbindungen besitzen pharmakologische Eigenschaften, die in mehreren Veröffentlichungen über Forskolin beschrieben worden sind. Die Verfahren zur Prüfung der verschiedenen pharmakologischen Eigenschaften sind in den erwähnten Veröffentlichungen über Forskolin beschrieben worden.

Die erfindungsgemäß erhaltenen Verbindungen eignen sich zur Behandlung von Glaukom, Hypertonie, Herzinsuffizienz, Entzündung, Allergien und Bronchialerkrankungen sowie Erkrankungen des Immunsystems.

Die erfindungsgemäß herstellbaren Labdan-Derivate können oral, beispielsweise mit einem inerten Verdünnungsmittel oder mit einem eßbaren Träger, verabreicht werden. Sie können in Gelatinekapseln eingeschlossen oder zu Tabletten verpreßt werden. Zum Zwecke einer oralen therapeutißchen Verabreichung können die obigen Verbindungen mit Hilfs- und Trägerstoffen vermischt werden und in Form von Tabletten, Pastillen, Kapseln, Elixieren, Suspensionen, Sirup, Waffeln, Kaugummi usw. verwendet werden. Diese Zubereitungen sollten wenigstens 0,5 % der erfindungsgemäßen Verbindungen enthalten, jedoch kann dieser Gehalt in Abhängigkeit von der jeweiligen Form auch zwischen 4 % und 70 % des Gewichtes der Einheit schwanken. Die Menge der erfindungsgemäßen Verbindung in solchen Zubereitungen sollte so bemessen sein, daß eine entsprechende Dosierung ermöglicht wird. Bevorzugte Zubereitungen werden so hergestellt, daß eine orale Dosiseinheitsforn zwischen 0,5 und 10 mg/kg Körpergewicht der erfindungsgemäß erhaltenen Verbindung enthält.

Die Tabletten, Pillen, Kapseln, Pastillen usw. können weiterhin die folgenden Stoffe enthalten: Bindemittel wie mikrokristalline Cellulose, Tragantgummi oder Gelatine; Hilfs- und Trägerstoffe die Stärke oder Milchzucker, Zerfallmittel wie Alginsäure, Maisstärke usw.; Gleitmittel vie Magnesiumstearat oder kolloidales Siliciumdioxid ; Süßstoffe wie Rohrzucker oder Saccharin oder Geschmacksstoffe wie Pfefferminze, Methylsalicylat oder Orangenaroma. Wnne die Dosiseinheit eine Kapsel ist, kann sie noch zusätzlich in obigen Substanzen flüssige Träger wie Fettöl enthalten. Andere Dosiseinheiten können weitere Substanzen enthalten, die die physische Form der Dosiseinheit verändern, beispeilsweise Überzüge. So können Tabletten oder Pillen mit Zucker, Schellack oder anderen enterischen Überzugsmittel überzogen sein. Ein Sirup kann zusätzlich Rohrzucker als Süßstoff und bestimmte Konservierungsstoffe, Farbstoffe und Aromatstoffe enthalten. Die bei der Herstellung dieser verschiedenen Zubereitungen verwendeten Stoffe sollten pharmazeutisch rein und in den verwendeten Menge nicht toxisch sein.

Zum Zwecke der parenteralen therapeutischen Verabreichung können die erfindugsgemäß erhältlichen Labdan-Derivate in eine Lösung oder Suspension gebracht werden. Solche Zubereitungen sollten wenigstens 0,1 % der obigen Verbindung enthalten, jedoch kann dieser Gehalt zwischen 0,5 und ca. 30 % des Gewichts der Zubereitung schwanken. Die Menge der vorliegenden Verbindung in diesen Zubereitungen sollte so bemessen sein, daß eine entsprechende Dosierung erreicht wird. Bevorzugte Zubereitungen werden so hergestellt, daß eine parenterale Dosiseinheit zwischen 0,05 und 10 mg/kg Körpergewicht der erfindungsgemäß erhaltenen Verbindung enthält.

Die Lösung und Suspension können weiterhin die folgenden Komponenten enthalten: sterile Verdünndungsmittel wie Wasser zur Injektion, Salzlösung, gebundene Öle, Polyäthylenglykole, Glyzerin, Propylenglykol oder andere synthetische Lösungsmittel; antibakteriell wirksame Mittel wie Benzylalkohol oder Methylparabene; Antioxidantien wie Ascorbinsäure oder Natriumbisulfit; Geliermittel wie Äthylendiamintetraessigsäure; Puffer wie Acetate, Citrate oder Phosphate sowie Mittel zur Regulierung des osmotischen Druckes, wie Natriumchlorid oder Dextrose. Die parenterale Zubereitung kann in Ampullen, Einwegspritzen oder Mehrfachdosisampullen aus Glas oder Plastik eingeschweißt sein.

Die nachfolgenden Beispiele sollen die Erfindung illlustrieren.

Allgemeines Verfahren nur mikrobiellen Transformation und Isolierung der Substanzen

100 ml einer Nährlösung (Sabouraud- oder Czapek-Dox-Medium (Difco Manual, 9. Aufl., 1969), dem Hefeextrakt (0,02 bis 0,2 %), Maisquellrasser (0,2 - 1,0 %) und Spurenelemente zugesetzt worden sind, werden in einen 500 ml-Erlenmeyer-Kolben gefüllt und 20 Minuten lang bei 120°C sterilisiert. Das sterilisierte, abgekühlte Medium wird mit einer Sporensuspension des Mikroorganismus inokuliert. Nach 48 Stunden Wachstum bei 28°C - 30°C auf einem Drehschüttler mit 220 Umdrehungen/Min., 4,8 cm Hub, werden 5 ml in 100 ml des gleichen sterilisierten Mediums inokuliert und dieses weitere 8 bis 10 Tage wachsen gelassen. Das Substrat (200 mg/ 100 ml Nährlösung) wird in Methanol (0,5 ml bis 1 ml) gelöst, durch gesinterten Glasfilter vorsterilisiert (G-5, Corning) und jedem Kolben entweder gleichzeitig mit der Vorkultur-Inokulation oder nach 36 bis 48 Stunden Wachstum zugesetzt. Das Substrat wird vorzugsweise nach 48 Stunden zugesetzt. Die Gesamtinkubationsdauer beträgt 8 bis 10 Tage, dann werden die Kolbeninhalte zusammengeschüttet und das Kulturbrühenfiltrat durch Filtration von der Mycelmasse spariert. Die Brühe und Mycelmasse werden getrennt mit organischen Lösungsmitteln wie Äthylacetat, Chloroform wird Methylenchlorid extrahiert. Als Lösungsmittel wird Chloroform bevorzugt. Die kombinierten Extrakte werden mit Wasser gewaschen, über Trocknungsmitteln wie Natriumsulfat getrocknet und dann im Vakuum zu einem Rückstand konzentriert, der

durch Chromatographie auf einem geeigneten Adsorbens und Kristallisation gereinigt wird. Als Adsorbens wird Kieselgel bevorzugt.

Als Medium wird das Sabouraud-Medium mit 0,2 % Hefeextrakt und 0,2 % Maisbrühe bevorzugt. Die bevorzugte Temperatur liegt bei 28°C, die bevorzugte Erntezeit liegt bei 8 Tagen.

**Beispiel 1**

Nach dem allgemeinen, oben beschriebenen Verfahren wurde unter Verwendung des Substrats 7-Deacetyl-1,9-Didesoxy-forskolin und des mikrobiellen Stammes H/134 die Substanz 7-Deacetylforskolin mit einem Schmelzpunkt von 177 - 78°C gewonnen.

**Beispiel 2**

Nach dem allgemeinen, oben beschriebenen Verfahren wurde unter Verwendung des Substrats 7-Deacetylforskolin und des mikrobiellen Stammes FF/406 die Substanz 3β-Acetoxy-7-deacetylforskolin mit einem Schmelzpunkt von 256 - 88°C gewonnen.

**Beispiel 3**

Nach dem allgemeinen, oben beschriebenen Verfahren wurde unter Verwendung ses Substrats 1,9-Didesoxyforskolin und des mikrobiellen Stammes FF/172 die Substanz 7-Deacetyl-2α-hydroxy-1,9-didesoxyforskolin mit einem Schmelzpunkt von 86 - 88°C gewonnen.

**Beispiel 4**

Nach dem allgemeinen, oben beschriebenen Verfahren wurde unter Verwendung des Substrats 1,9-Didesoxyforskolin und des mikrobiellen Stanmes FF/101 die Substanz 2α-Hydroxy-1,9-didesoxyforskolin mit einem Schmelzpunkt von 209 - 211°C gewonnen.

**Beispiel 5**

Nach dem allgemeinen, oben beschriebenen Verfahren wurde unter Verwendung des Substrats 1,9-Didesoxyforskolin und des mikrobiellen Stemmes H/134 die Substanz 7-Deacetyl-2β-hydroxy-1,9-didesoxyforskolin als feste Substanz mit einem Schmelzpunkt von 84 - 86°C gewonnen.

**Beispiel 6**

Nach dem allgemeinen, oben beschriebenen Verfahren wurde unter Verwendung des Substrats 1,9-Didesoxyforskolin und des mikrobiellen Stammes FF/101 die Substanz 2β-Hydroxy-1,9-didesoxyforskolin mit einem Schmelzpunkt von 258 - 261°C gewonnen.

**Beispiel 7**

Nach dem allgemeinen, oben beschriebenen Verfahren wurde unter Verwendung des Substrats 1,9-Didesoxyforskolin und des mikrobiellen Stammes H/134 die Substanz 6β-Acetyl-7-deacetyl-2β-hydroxy-1,9-didesoxyforskolin mit einem Schmelzpunkt von 168 - 170°C gewonnen.

**Beispiel 8**

Nach dem allgemeinen, oben beschriebenen Verfahren wurde unter Verwendung des Substrats 1,9-Didesoxyforskolin und des mikrobielllen Stammes FF/173 die Substanz 3β-Hydroxy-1,9-didesoxyforskolin mit einem Schmelzpunkt von 271 - 272°C gewonnen.

**Beispeil 9**

Nach dem allgemeinen, oben beschriebenen Verfahren wurde unter Verwendung des Substrats 1,9-Didesoxyforskolin und des mikrobiellen Stammes FF/383 due Substanz 7-Deacetyl-1,9-didesoxyforskolin mit einem Schmelzpunkt von 78 - 80° C gewonnen.

**Beispiel 10**

Nach dem allgemeinen, oben beschriebenen Verfahren wurde unter Verwendung des Substrats 1,9-Didesoxyforskolin und des mikrobiellen Stammes FF/695 die Substanz 6β-Acetyl-7-deacetyl-3β-hydroxy-1,9-didesoxyforskolin mit einem Schmelzpunkt von 200 - 202° C gewonnen.

**Beispiel 11**

Nach dem allgemeinen, oben beschriebenen Verfahren wurde unter Verwendung des Substrats 1,9-Didesoxyforskolin und des mikrobiellen Stammes FF/93 die Substanz 7-Deacetyl-1,9-didesoxyforskolin mit einem Schmelzpunkt von 121 - 122° C gewonnen.

**Beispiel 12**

Nach dem allgemeinen, oben beschriebenen Verfahren wurde unter Verwendung des Substrats 1,9-Didesoxyforskolin und des mikrobiellen Stammes T-8252904 die Substanz 6β-Acetyl-7-deacetyl-1,9-didesoxyforskolin mit einem Schmelzpunkt von 149 - 150° C gewonnen.

**Beispiel 13**

Nach dem allgemeinen oben beschriebenen Verfahren wurde unter Verwendung des Substrats 1,9-didesoxyforskolin und des mikrobiellen Stammes H/1 die Substanz 3β-14,15-trihydroxy-1,9-didesoxyforskolin in Form eines dicken, viskosen Öls gewonnen.

**Patentanspruch**

1. Verfahren zur Herstellung von Forskolinderivaten, dadurch gekennzeichnet, daß man auf ein bestimmtes Forskolinderivat als Substrat in einem Nährmedium auf übliche Weise einen bestimmten Mikroorganismus einwirken läßt und die gebildete Verbindung nach Beendigung der Fermentation aus der Kulturbrühe und dem Mycel durch Extraktion mit einem organischen Lösungsmittel und anschließende Chromatographie und/oder Kristallisation isoliert, wobei man

a) aus der Verbindung der Formel III

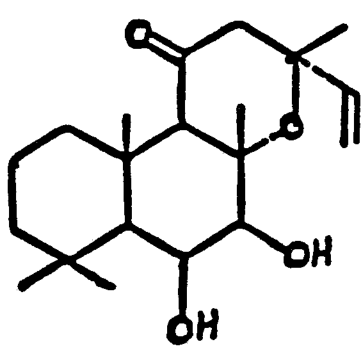

III

als Substrat und unter Verwendung des Stammes H/134 (DSM 3205) die Verbindung der Formel IV

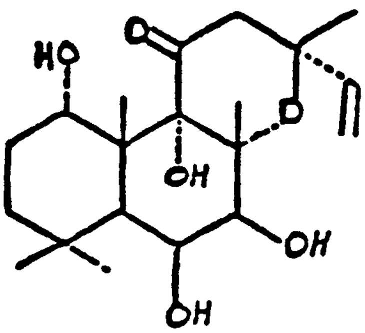

IV

erhält,

b) aus der Verbindung der Formel IV als Substrat und unter Verwendung des Stammes FF/406 (DSM 3211) die Verbindung der Formel V

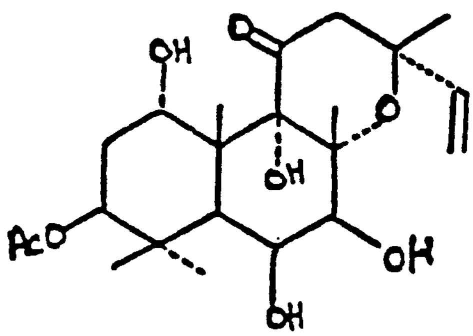

V

worin Ac den Rest $CH_3CO$- bedeutet, erhält,

c) aus der Verbindung der Formel VI

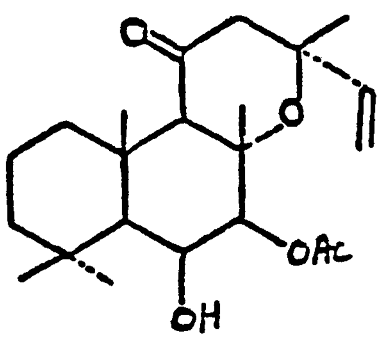

VI

worin Ac den Rest $CH_3CO$- bedeutet als Substrat und

$c_1$) unter Verwendung des Stammes FF/172 (DSM 3208) die Verbindung der Formel VII

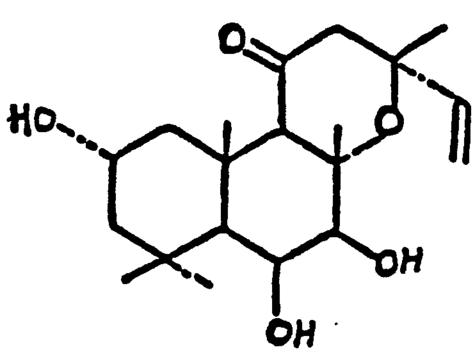

VII

oder

$c_2$) unter Verwendung des Stammes FF/101 (DSM 3207) die Verbindung der Formel VIII

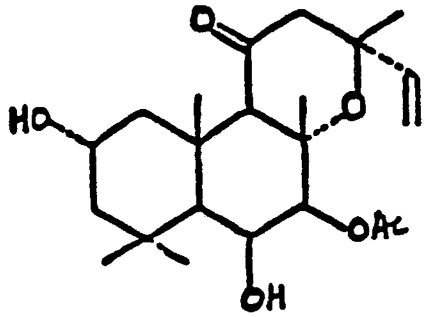

VIII

worin Ac den Rest $CH_3CO$- bedeutet,
oder
$c_3$) unter Verwendung des Stammes H/134 (DSM 3205) die Verbindung der Formel IX

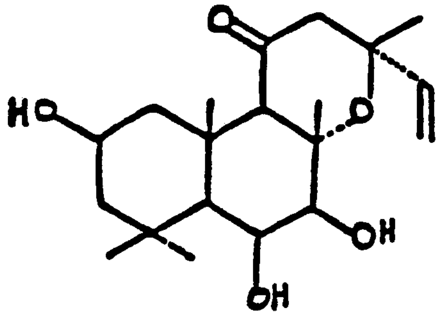

IX

oder
$c_4$) unter Verwendung des Stammes FF/101 (DSM 3207) die Verbindung der Formel X

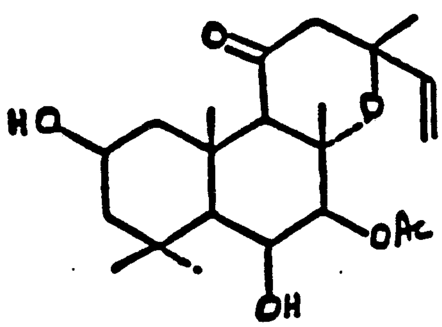

X

worin Ac den Rest $CH_3CO$- bedeutet,
oder
$c_5$) unter Verwendung des Stammes H/134 (DSM 3205) die Verbindung der Formel XI

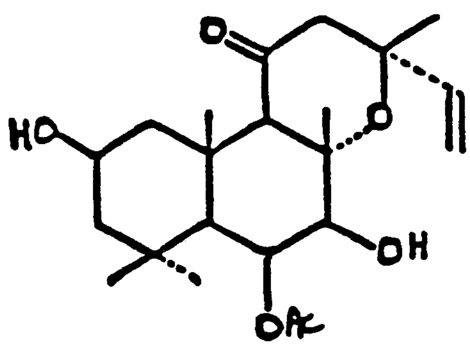

XI

worin Ac den Rest $CH_3CO$- bedeutet,
oder

$c_6$) unter Verwendung des Stammes FF/173 (DSM 3209) oder H/1 (DSM 3202) die Verbindung der Formel XII

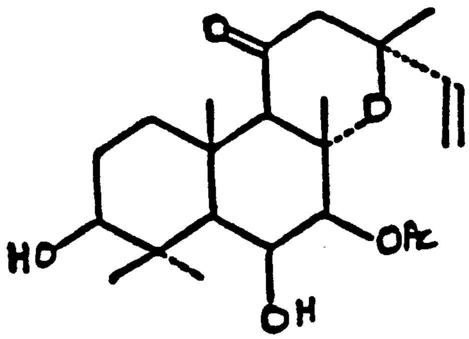

XII

worin Ac den Rest $CH_3CO$- bedeutet,
oder
$c_7$) unter Verwendung des Stammes FF/383 (DSM 3210) die Verbindung der Formel XIII

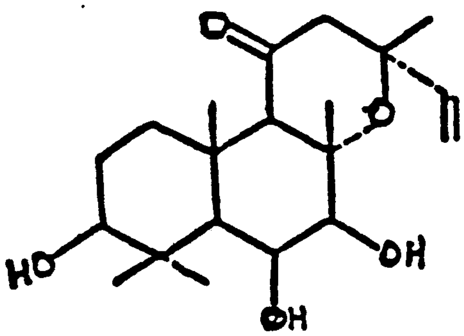

XIII

oder
$c_8$) unter Verwendung des Stammes FF/695 (DSM 3212) die Verbindung der Formel XIV

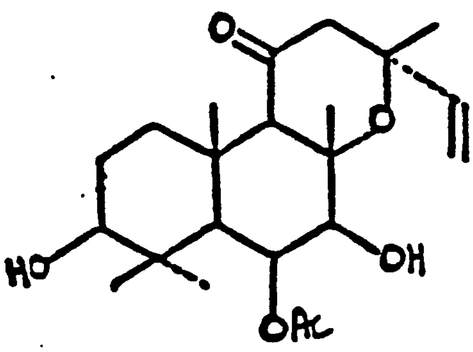

XIV

worin Ac den Rest $CH_3CO$- bedeutet,
oder
$c_9$) Unter Verwendung des Stammes FF/93 (DSM 3206) oder FF/172 (DSM 3208) die Verbindung der Formel XV

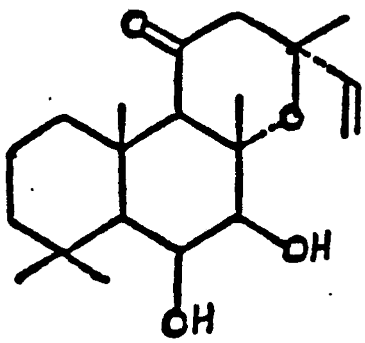

XV

oder

$c_{10}$) unter Verwendung des Stammes H/3 (DSM 3203) oder H/19 (DSM 3204) oder Y-82 52 904 (DSM 3213) die Verbindung der Formel XVI

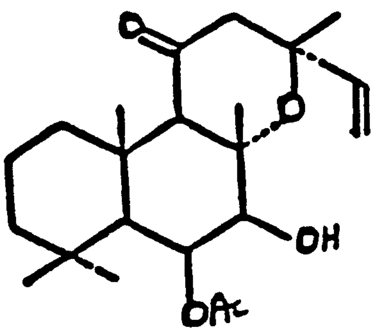

XVI

worin Ac den Rest $CH_3CO$- bedeutet,
oder

$c_{11}$) unter Verwendung des Stammes H/1 (DSM 3202) die Verbindung der Formel XVII

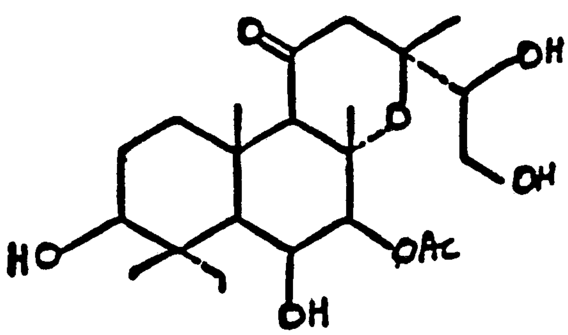

XVII

worin Ac den Rest $CH_3CO$- bedeutet, erhält.

**Claim**

1. A process for the preparation of forskolin derivatives, which comprises a particular microorganism being allowed to act on a particular forskolin derivative as substrate in a nutrient medium in a customary manner and, after completion of the fermentation, the compound which has formed being isolated from the culture broth and the mycelium by extraction with an organic solvent and subsequent chromatography and/or crystallization, where

a) with the compound of the formula III

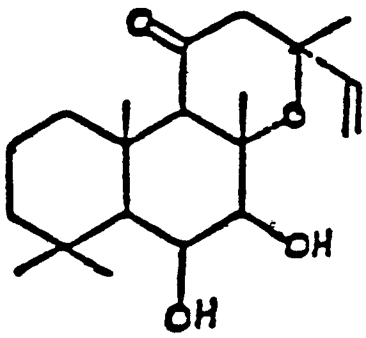

III

as substrate and using the strain H/134 (DSM 3205), there is obtained the compound of the formula IV

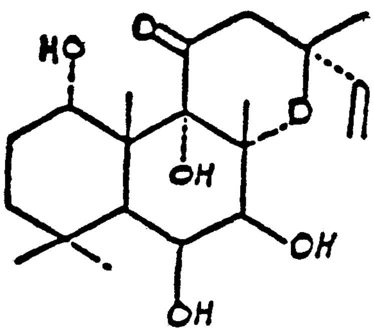

IV

b) with the compound of the formula IV as substrate and using the strain FF/406 (DSM 3211), there is obtained the compound of the formula V

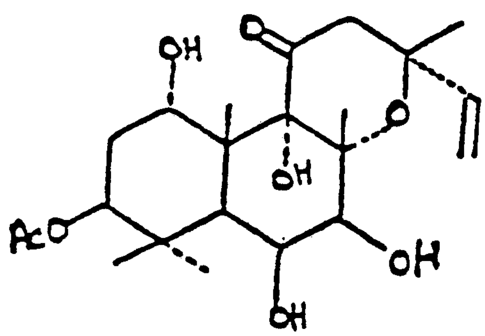

V

in which Ac denotes the radical $CH_3CO$-,
c) with the compound of the formula VI

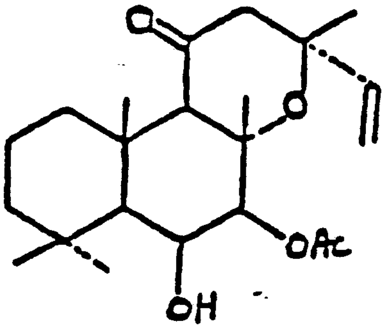

VI

in which Ac denotes the radical $CH_3CO$-, as substrate and
$c_1$ ) using the strain FF/172 (DSM 3208), there is obtained the compound of the formula VII

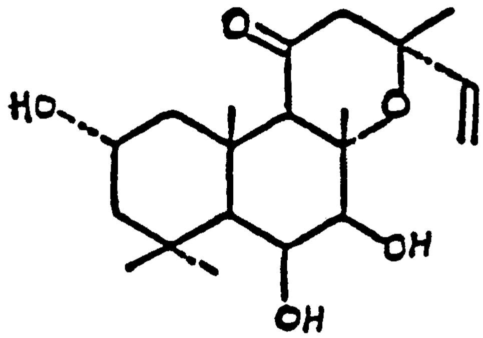

VII

or

$c_2$) using the strain FF/101 (DSM 3207), there is obtained the compound of the formula VIII

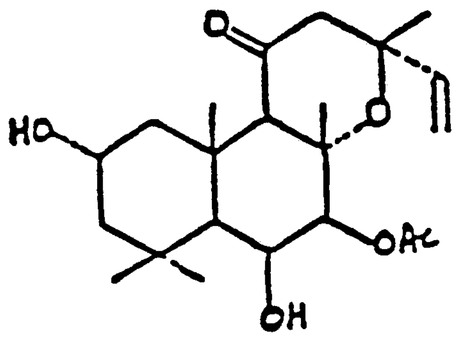

VIII

in which Ac denotes the radical $CH_3CO$-,

or

$c_3$) using the strain H/134 (DSM 3205), there is obtained the compound of the formula IX

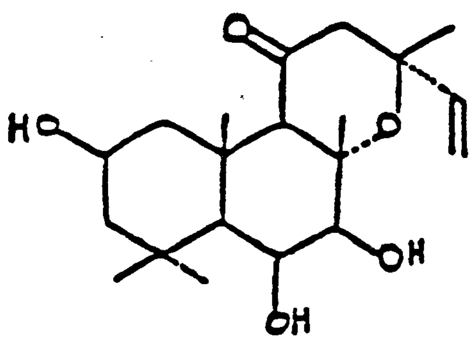

IX

or

$c_4$) using the strain FF/101 (DSM 3207), there is obtained the compound of the formula X

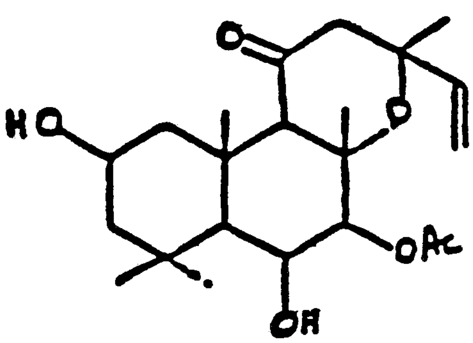

X

in which Ac denotes the radical $CH_3CO$-,

or

$c_5$) using the strain H/134 (DSM 3205), there is obtained the compound for the formula XI

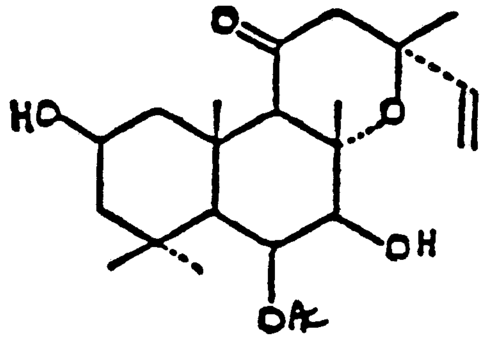

XI

in which Ac denotes the radical $CH_3CO$-,
or
$c_6$) using the strain FF/173 (DSM 3209) or H/1 (DSM 3202), there is obtained the compound of the formula XII

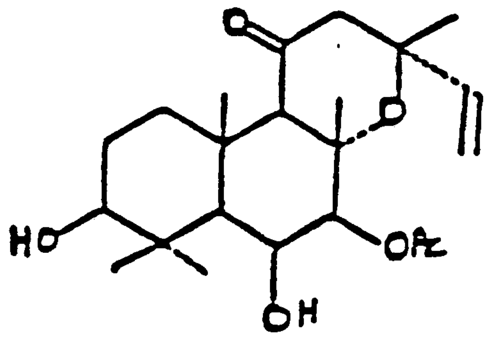

XII

in which Ac denotes the radical $CH_3CO$-,
or
$c_8$) using the strain FF/383 (DSM 3210), there is obtained the compound of the formula XIII

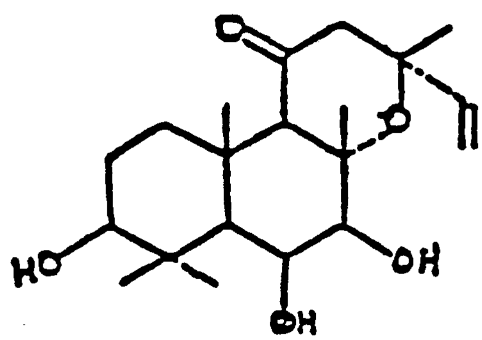

XIII

or
$c_8$) using the strain FF/695 (DSM 3212), there is obtained the compound of the formula XIV

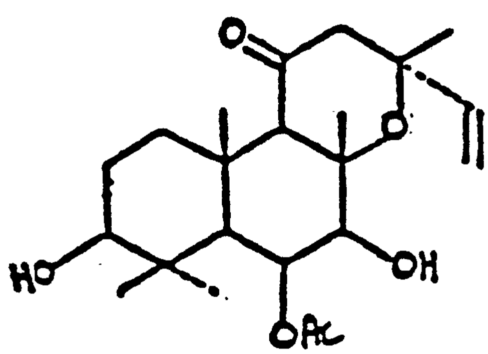

XIV

in which Ac denotes the radical $CH_3CO$-,
or

$c_9$) using the strain FF/93 (DSM 3206) or FF/172 (DSM 3208), there is obtained the compound of the formula XV

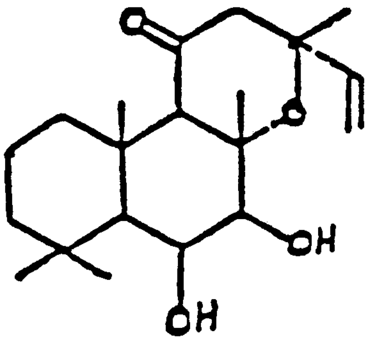

XV

or

$c_{10}$) using the strain H/3 (DSM 3203) or H/19 (DSM 3204) or Y-82 52 904 (DSM 3213), there is obtained the compound of the formula XVI

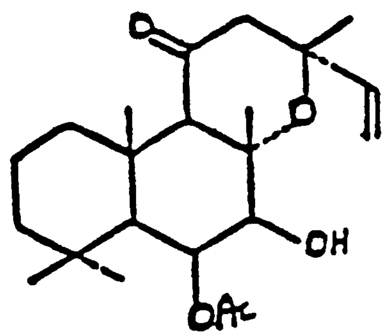

XVI

in which Ac denotes the radical $CH_3CO$-,

or

$c_{11}$) using the strain H/1 (DSM 3202), there is obtained the compound of the formula XVII

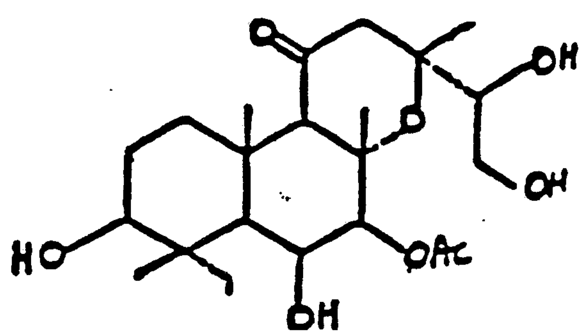

XVII

in which Ac denotes the radical $CH_3CO$-.

**Revendication**

1. Procédé pour la préparation de dérivés de la forskoline, caractérisé en ce que l'on fait agir à la manière habituelle un micro-organisme déterminé sur un dérivé déterminé de la forskoline en tant que substrat, dans un milieu nutritif, et une fois la fermentation terminée, on isole le composé formé, à partir du milieu de culture et hors du mycélium, par extraction avec un solvant organique et chromatographie et/ou cristallisation subséquentes, ce par quoi

a) à partir du composé de formule III

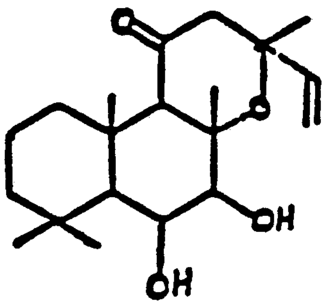

III

en tant que substrat et en utilisant la souche H/134 (DSM 3205), on obtient le composé de formule IV

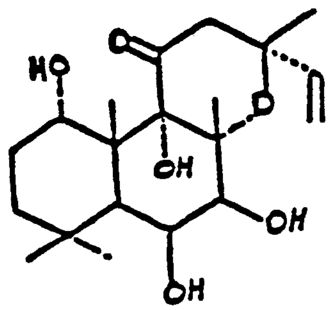

IV

b) à partir du composé de formule IV en tant que substrat et en utilisant la souche FF/406 (DSM 3211), on obtient le composé de formule V

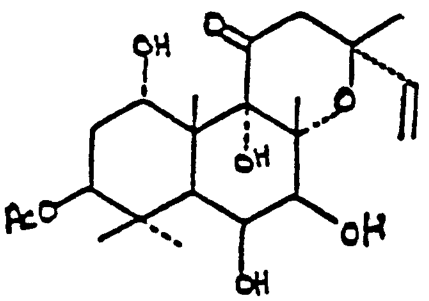

V

dans laquelle Ac représente le radical $CH_3CO$-,
c) à partir du composé de formule VI

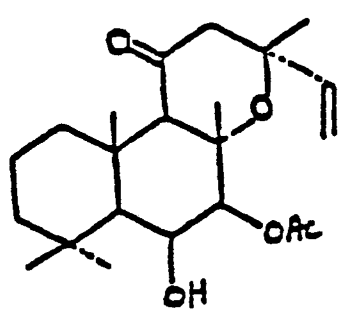

VI

dans laquelle Ac représente le radical $CH_3CO$-, en tant que substrat, et

$c_1$) en utilisant la souche FF/172 (DSM 3208), on obtient le composé de formule VII

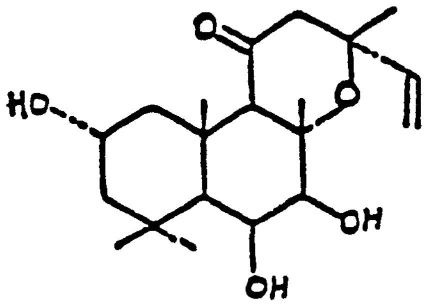

VII

ou

$c_2$) en utilisant la souche FF/101 (DSM 3207) , on obtient le composé de formule VIII

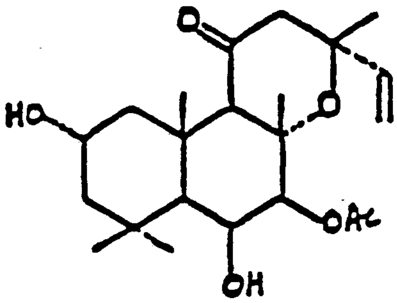

VIII

dans laquelle Ac représente le radical $CH_3CO$-,

ou

$c_3$) en utilisant la souche H/134 (DSM 3205), on obtient le composé de formule IX

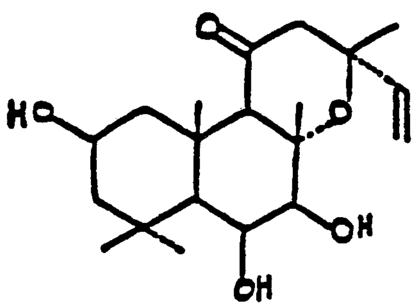

IX

ou

$c_4$) en utilisant la souche FF/101 (DSM 3207), on obtient le composé de formule X

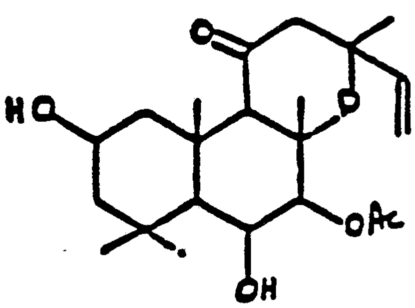

X

dans laquelle Ac représente le radical $CH_3CO$-,

ou

$c_5$) en utilisant la souche H/134 (DSM 3205), on obtient le composé de formule XI

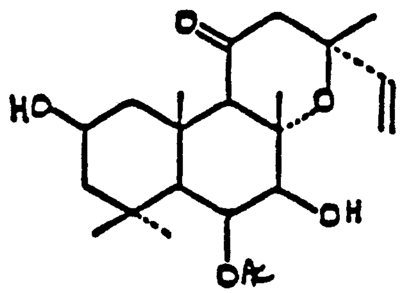

XI

dans laquelle Ac représente le radical $CH_3CO$-,
ou
$c_6$) en utilisant la souche FF/173 (DSM 3209) ou H/1 (DSM 3202), on obtient le composé de formule XII

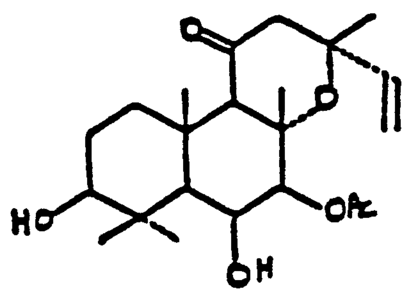

XII

dans laquelle Ac représente le radical $CH_3CO$-,
ou
$c_7$) en utilisant la souche FF/383 (DSM 3210), on obtient le composé de formule XIII

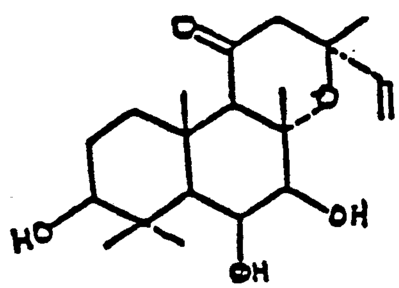

XIII

ou
$c_8$) en utilisant la souche FF/695 (DSM 3212), on obtient le composé de formule XIV

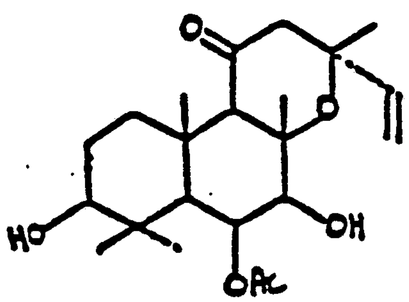

XIV

dans laquelle Ac représente le radical $CH_3CO$-,
ou

$c_9$) en utilisant la souche FF/93 (OSM 3206) ou FF/172 (DSM 3208), on obtient le composé de formule XV

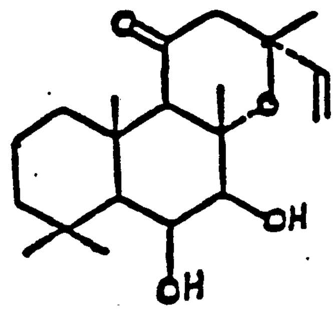

XV

ou

$c_{10}$) en utilisant la souche H/3 (DSM 3203) ou H/19 (DSM 3204) ou Y-82 52 904 (DSM 3213), on obtient le composé de formule XVI

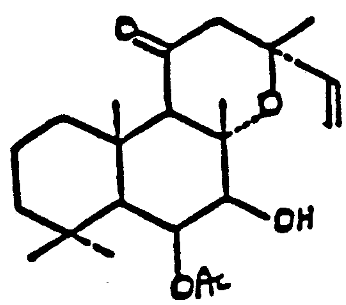

XVI

dans laquelle Ac représente le radical $CH_3CO$-,

ou

$c_{11}$) en utilisant la souche H/1 (DSM 3202), on obtient le composé de formule XVII

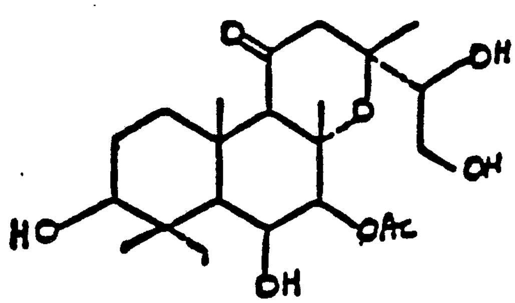

XVII

dans laquelle Ac représente le radical $CH_3CO$-.

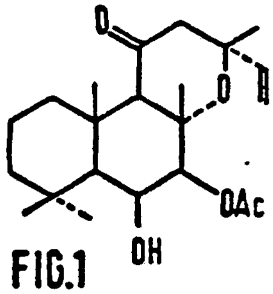
FIG.1

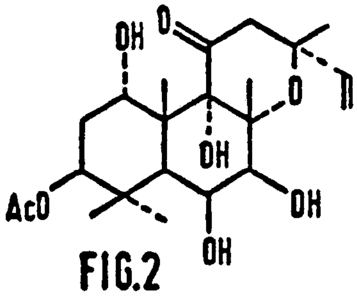
FIG.2

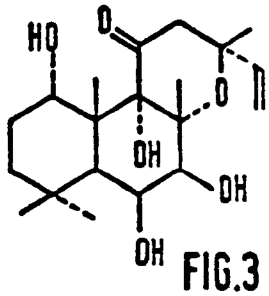
FIG.3

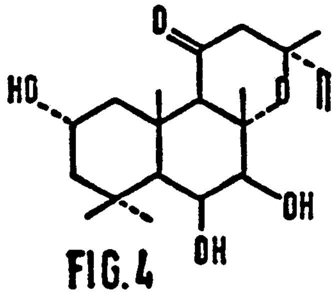
FIG.4

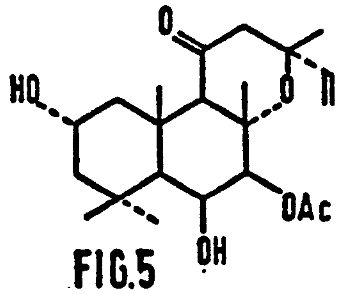
FIG.5

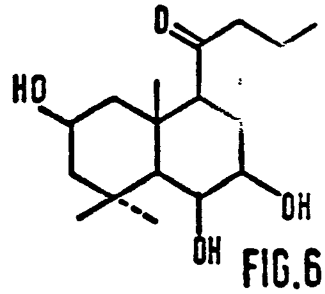
FIG.6

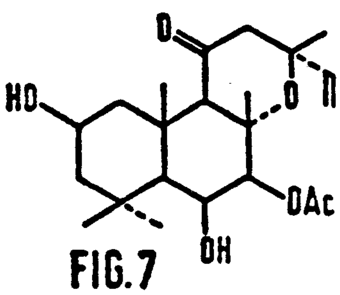
FIG.7

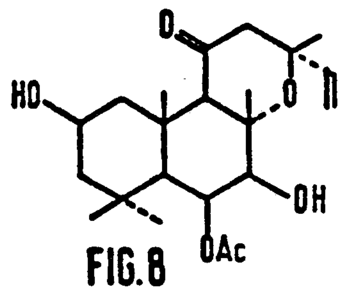
FIG.8

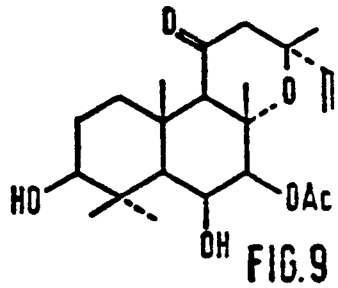
FIG.9

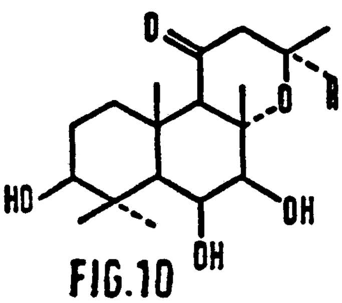
FIG.10

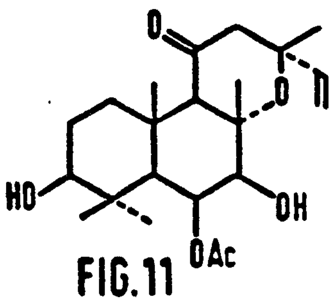
FIG.11

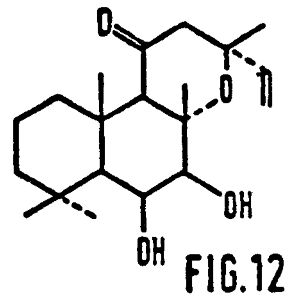
FIG.12

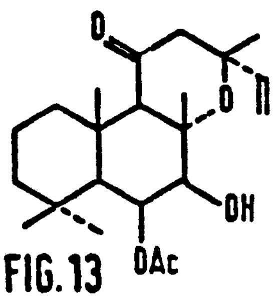
FIG.13

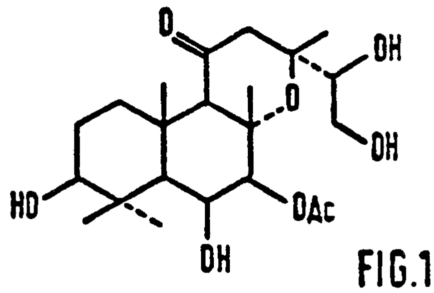
FIG.14